# EUROPEAN PATENT APPLICATION

(11) **EP 2 198 811 A1**
(43) Date of publication of application: **23.06.2010**
(21) Application number: 08791285.3
(22) Date of filing: 17.07.2008
(51) Int. Cl.: A61F 2/82

(54) **STENT PLACEMENT DEVICE AND STENT PLACEMENT METHOD**

(30) Priority: 01.09.2007 JP 2007227218
(71) Applicant: Sapporo Medical University, Hokkaido 060-0061 (JP); National University Corporation University of Fukui, Fukui-shi, Fukui 910-8507 (JP)
(72) Inventor: HYODOH, Hideki, Sapporo-shi Hokkaido 060-8556 (JP); SAKAI, Toyohiko, Yoshida-gun Fukui 910-1193 (JP)
(74) Representative: Niizuma, Yo
(86) International application number: PCT/JP2008/062931
(87) International publication number: WO 2009/028272

(57) **Abstract**

A stent placement device and a stent placement method, despite a simple structure thereof, can surely and readily deploy a stent by placing a distal end thereof prior to a proximal end thereof, having favorably low invasiveness, and smoothly insert and arrange the stent into a desired position, without damaging an internal lumen inner wall. A placement device 1 for deploying a stent 2 in an internal lumen includes a guide wire 3 which guides said stent 2 into a desired position in the internal lumen, a proximal tip 4 which is fixed at an intermediate position of said guide wire 3 and provided with a stent anchoring portion for anchoring said stent 2, and a stent converging and retaining member 5 which converges and retains a stent proximal end opening portion 2a of said stent 2.

## Description

### TECHNICAL FIELD

The present invention relates to a device for deploying a stent in an internal lumen, more particularly to a stent placement device capable of surely and readily deploying a stent by placing a distal end thereof prior to a proximal end thereof, having favorably low invasiveness, and smoothly inserting and arranging the stent into a desired position, and a stent placement method using the same.

### BACKGROUND ART

Conventionally, aortic aneurysm, such as dissociative aortic aneurysm, is treated by vascular prosthetics by thoracotomy and laparotomy. However, this conventional treatment involves operations having much burden on a patient's body due to highly-invasive arrangement of heart-lung machines for a specific affected site and hypothermia. Thus, this treatment cannot provide favorable effects in cases where weaker physical strength is found in an elderly patient, respiratory failures are caused due to excessive smoking habits, malignant tumors to be treated are found, complication is developed and further surgeries are difficult from previous records of thoracotomy or laparotomy.

Other than said vascular prosthetics, stent treatment methods have been introduced in recent years. Specifically, a stent is inserted into a femoral artery to lead to an affected site and then adhered to a blood vessel from a lumen to be deployed. These methods can prevent rupture of a blood vessel by interrupting blood flow leading to aneurysm to reduce blood pressure, using a new blood vessel cavity provided around an aneurysm.

With no thoracotomy or laparotomy, in addition to low invasiveness such as minimum incision and a significantly low burden on a patient, these stent treatment methods are employed even in a case where vascular prosthetics cannot be applied. Also, a stent is used not only for treating said aortic aneurysm and dissociative aortic aneurysm, but also for expanding a narrowed or blocked lumen of a tubular organ in the body to prevent another constriction.

Conventionally, some stent placement devices for deploying a stent in an internal lumen are developed (Patent Documents 1 to 3).

For example, Patent Document 1 discloses a stent graft indwelling implant which is provided with a notch with which a hook mounted on a stent graft is detachably engaged and is capable of self-expanding the stent graft retained in a stent graft retaining portion at an indwelling position determined in an in vivo tubular body by engaging detachably the hook with the notch.

Patent Document 2 discloses a stent placement device capable of deploying a stent in an in vivo tubular body, the stent being arranged to the outer periphery of an elastic resin coating part of a tip part of a flexible shaft and housed with reduced in diameter inside a sheath, by pulling a sheath toward the base part from the flexible shaft to smoothly discharge the stent from the tip part of the sheath.

Patent Document 3 discloses a stent graft placement device which retains a stent graft deployed by making the stent graft contracted and releasing a distal end of the stent graft prior to a proximal end thereof.

### PRIOR ART DOCUMENTS

### Patent Document

Patent Document 1: Japanese Unexamined Patent Application publication No. 2000-350785
Patent Document 2: Japanese Unexamined Patent Application publication No. 2005-58458
Patent Document 3: Japanese Unexamined Patent Application publication (Translation of PCT Application) No. 2006-501890

### SUMMARY OF THE INVENTION

### PROBLEMS TO BE SOLVED BY THE INVENTION

However, in the stent placement devices disclosed in Patent Documents 1 and 2, a stent is deployed by its gradual release from a proximal end thereof to a distal end thereof. Particularly in disorders such as thoracic aneurysm and abdominal aneurysm, a stent to be deployed is released according to a direction of blood flow, resulting in position shift of the stent from a desired position. After a guide wire is inserted into an internal lumen, a stent placement device must be additionally inserted thereinto, thereby requiring plural operations for inserting the stent against blood flow and thus causing burden on a patient and damaging a blood vessel.

Moreover, in the stent placement devices disclosed in Patent Documents 1 and 2, a stent placement device and a guide wire are separately operated to make difficult delicate control operation of a stent. In addition, a bent section in a stent insertion step or a stent deploying part causes rotation or torsion of a stent placement device, fails to adjust a stent placement position or direction, or position shift of the stent when an unstable sheath is withdrawn at a stent deploying part. Also, a proximal tip comprising a stent placement device cannot be collected and it will be left in the body.

Additionally, the stent placement devices disclosed in Patent Documents 1, 2 and 3 comprise a double structure of a sheath which retains a stent, a catheter or elastomer resin, resulting in the following problems: (1) a stent placement device fails to smoothly come to a desired position due to interference with a bent section from a larger diameter thereof, (2) a sheath cannot be withdrawn from the bent section where the stent is deployed and (3) an internal lumen inner wall is damaged by snagging.

The stent graft placement device disclosed in Patent Document 3 must be controlled and operated only on a proximal side, because a guide wire and a catheter don't extend further from a proximal end of the stent graft placement device. Consequently, a stent graft which is arranged at a desired position cannot eliminate rotation or torsion thereof at a bent section, resulting in difficulty in delicate stent control and operation. Also, in a case where a stent graft placement device is inserted against blood flow, delicate stent control and operation become more difficult.

In the stent graft placement device disclosed in Patent Document 3, a proximal end of a stent graft is retained by an anchoring loop operated by a trigger wire and a distal end is retained by a capsule and a trigger wire, thereby causing uneven force distribution on the stent graft when the trigger wire is withdrawn and position shift of the stent graft from a desired position.

Moreover, in the stent graft placement device disclosed in Patent Document 3, when a distal end of the stent graft is released, a proximal end of the stent graft is in a significantly unstable state, because it is retained only by a trigger wire, not by the stent graft placement device. Then, when the stent graft proximal end retained is released by the trigger wire, the stent graft is shifted from a desired position.

Also, the stent graft placement device disclosed in Patent Document 3 requires technical skills due to more complicated deploying operations in a device composed of many components such as a handle, release mechanism and screws, insufficient operating precision and speed and more operational errors. In particular, more specific skills are required to deploy a stent at a desired position having no stent position shift, in cases where an allowance in a placement position is small and a blood flow is large.

It is, therefore, one object of the present invention to provide a stent placement device and a stent placement method, despite a simple structure thereof, which is capable of surely and readily deploying a stent by placing a distal end thereof prior to a proximal end thereof, having favorably low invasiveness, and smoothly inserting and arranging the stent into a desired position, without damaging an internal lumen inner wall.

### MEANS FOR SOLVING THE PROBLEM

A stent placement device according to the present invention, in a placement device for deploying a stent in an internal lumen, comprises a guide wire which guides said stent to a desired position in an internal lumen, a proximal tip which is fixed at an intermediate position of said guide wire and provided with a stent anchoring portion for anchoring said stent, and a stent converging and retaining member which converges and retains a stent proximal end opening portion of said stent.

According to an embodiment in this invention, said stent converging and retaining member may comprise an operation main body portion formed of a longitudinal thin wire and a proximal tip expanded portion composed of plural thin linear members which are radially and expandably branched at a proximal tip of the operation main body portion.

According to an embodiment in this invention, said stent converging and retaining member may be formed of a shape-memory material.

Said stent anchoring portion in this invention may be composed of one or plural anchor holes, and said proximal tip expanded portion of said stent converging and retaining member may be engaged with said anchor hole to be anchored.

Meanwhile, a stent placement method according to the present invention is a method for deploying a stent at a desired position in an internal lumen, comprising a stent arranging step for arranging a stent at a desired position by restraining and retaining a proximal end opening portion of a stent reduced in diameter by a stent converging and retaining member, anchoring the stent in a proximal tip fixed at an intermediate position of a guide wire and inserting the stent into an internal lumen with the stent being housed in a longitudinal tube-shaped sheath and guiding the stent by said proximal tip, a 1st stent deploying step for deploying only a stent distal end at a desired position by pulling said sheath toward a distal end to expand the distal end of said stent with a proximal end opening portion of said stent restrained and retained by said stent converging and retaining member, a 2nd stent deploying step for entirely deploying the stent at a desired position by releasing the distal end opening portion by said stent converging and retaining member and a step for collecting said proximal tip from the body by withdrawing said guide wire from an internal lumen.

### ADVANTAGEOUS EFFECT OF THE INVENTION

A stent placement device and a stent placement method according to the present invention, despite a simple structure thereof, can surely and readily deploy a stent by smoothly inserting and arranging the stent into a desired position, without damaging an internal lumen inner wall, and thereafter completely collect the stent placement device from the body.

### BRIEF DESCRIPTION OF THE DRAWINGS

Figure 1 is a perspective view showing an embodiment of a stent placement device according to the present invention.
Figure 2 is a longitudinal sectional view in Figure 1 of this embodiment.
Figure 3 is a diagram showing a guide wire and a proximal tip of this embodiment.
Figure 4 is a side view of a proximal tip rear end showing a guide wire and a proximal tip of this embodiment.
Figure 5 is a diagram showing a stent converging and retaining member of this embodiment, in which a proximal tip expanded portion is not expanded in (a) and a proximal tip expanded portion is expanded in (b).
Figure 6 is a diagram showing a proximal tip expanded portion of a stent converging and retaining member of this embodiment, in which a proximal tip expanded portion is not expanded in (a) and a proximal tip expanded portion is expanded in (b).
Figure 7 is an explanatory diagram showing a stent proximal end opening portion converged and retained by a stent converging and retaining member of this embodiment.
Figure 8 is an explanatory diagram showing a stent placement method of this embodiment, in which Step 1 is in (a), Step 2 in (b), Step 3 in (c) and Step 4 in (d).
Figure 9 is an explanatory diagram showing a stent placement method of this embodiment as a method for treating thoracic aneurysm, in which Step 1 is in (a), Step 2 in (b), Step 3 in (c) and Step 4 in (d).

### BEST MODE FOR CARRYING OUT THE INVENTION

An embodiment of a stent placement device and a stent placement method according to the present invention will be described with reference to the drawings.

First of all, an embodiment of a stent placement device according to the present invention will be described.

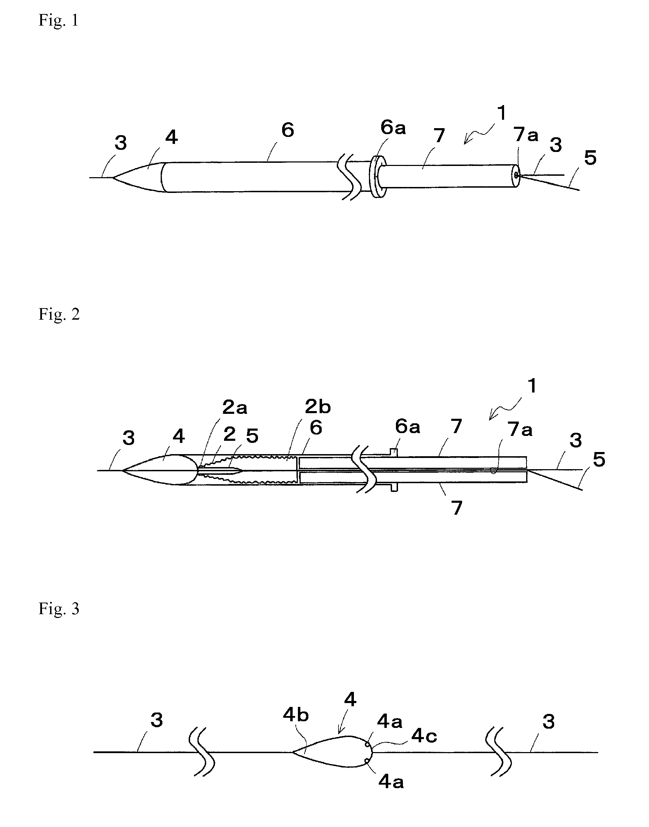

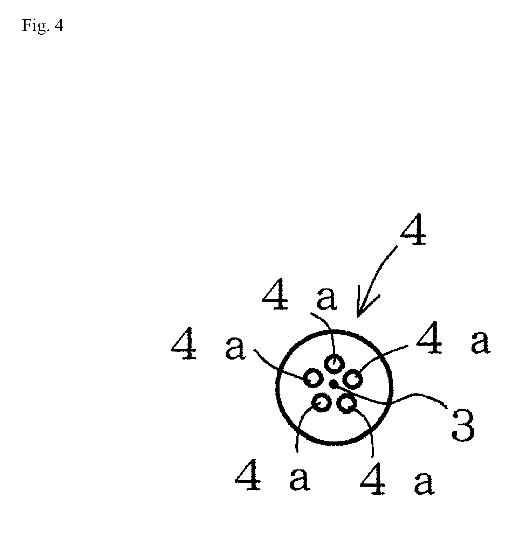

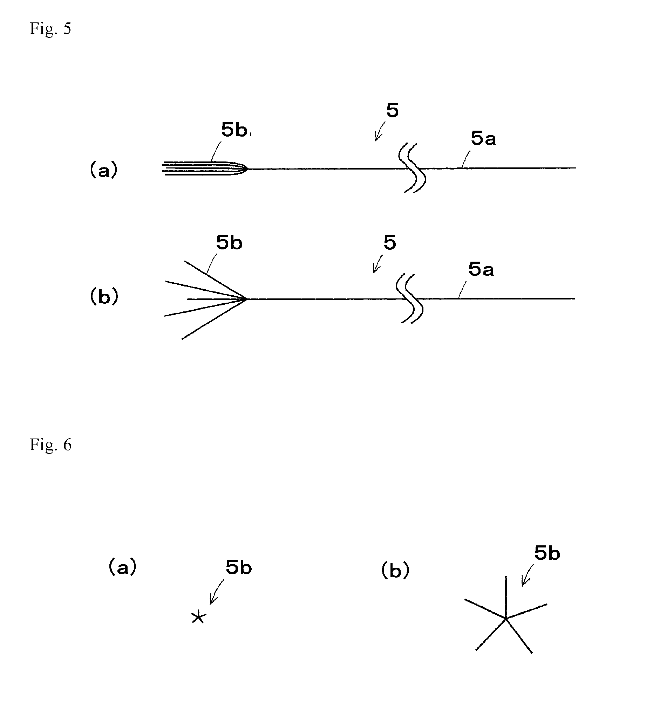

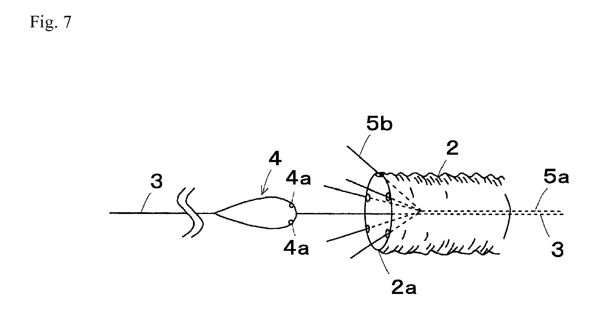

Figure 1 is a perspective view showing one embodiment of a stent placement device according to the present invention, Figure 2 is a longitudinal sectional view of Figure 1, Figures 3 and 4 are diagrams showing a guide wire and a proximal tip of this embodiment, Figures 5 and 6 are diagrams showing a stent converging and retaining member of this embodiment, and Figure 7 is an explanatory diagram showing a stent proximal end opening portion converged and retained by a stent converging and retaining member of this embodiment.

As shown in Figures 1 and 2, a stent placement device 1 of this embodiment comprises a stent 2, a guide wire 3, a proximal tip 4, a stent converging and retaining member 5, a sheath 6 and a pusher 7. As shown in Figure 2, the proximal tip 4 is fixed so that it is penetrated into the guide wire 3. As shown in Figure 2, the stent placement device 1 is provided with the proximal tip 4, followed by the stent 2 and the pusher 7 arranged downward from the proximal tip 4. The guide wire 3 and the stent converging and retaining member 5 pass through a lumen of the stent 2 and inserted into an insertion port 7a formed in a longitudinal nearly center of the pusher 7.

As shown in Figure 2, the stent 2 is anchored in the proximal tip 4 so that the stent proximal end opening portion 2a is converged and retained by the stent converging and retaining member 5. The proximal tip 4, the stent 2, part of the pusher 7, part of the guide wire 3 and part of the stent converging and retaining member 5 are integrally housed in the stent placement device 1.

Here, the stent 2 of this embodiment is not particularly limited as long as it is deployed in an internal lumen to stably provide a vascular lumen, e.g. a shape-retentive material which can be recovered from a diameter-reduced state, a flexible material and a shape-memory material. A shape-memory material is preferably Cu-Al-Ni alloy, Cu-Zn-Al alloy or Ni-Ti alloy such as nitinol.

Stents in this invention include a stent graft coated or sewn with a graft as an artificial blood vessel or drug-eluting stent, as well as ordinary stents such as a metal reticulate tube, and can be selected in accordance with an affected site in an internal lumen where a stent is deployed. A stent 2 of this embodiment is a stent graft.

Next, as shown in Figures 1 to 4, a guide wire 3 of this embodiment plays a role in guiding a proximal tip 4 and a stent 2 anchored therein to a desired position in an internal lumen. Specifically, after inserting the stent into the internal lumen, the stent 2 therein can be guided to a desired position by operating a guide wire 3 left in front of an insertion port and a guide wire 3 extended extracorporeally from an extraction port via a desired position.

Specifically, a guide wire 3 of this embodiment, which is inserted from an insertion port into an internal lumen and extended extracorporeally from an extraction port via a desired position again, is not particularly limited in length according to the present invention, but it may be extracorporeally operated a stent in an internal lumen.

A material of a guide wire according to the present invention is not particularly limited, but it is preferably Ni-Ti alloy such as nitinol which can smoothly insert an internal lumen inner wall into an internal lumen at a bent section without damaging the internal lumen inner wall.

Next, as shown in Figures 1 to 4, a proximal tip 4 of this embodiment, into which a guide wire 3 is penetrated, is fixed at an intermediate position to guide the stent 2 by cooperating with the guide wire 3. Thus, it is not necessary to arrange additional catheter in a stent placement device 1. One inserting operation is enough to insert the guide wire 3 and the proximal tip 4 into an internal lumen, and when the guide wire 3 is withdrawn after deploying the stent 2 at a desired position, it is possible to assuredly collect the proximal tip 4 from the body as well.

A proximal tip according to the present invention is not particularly limited in position to be fixed as long as it is not at both ends of the guide wire 3, but at an intermediate position therebetween. In this embodiment, a proximal tip 4 is fixed to the guide wire 3 so that a stent 2 in an internal lumen can be operated extracorporeally. Specifically, in order to extracorporeally operate the stent 2 in the internal lumen bi-directionally, one end of the guide wire 3 is left in front of an insertion port, and the other end is extended from an extraction port extracorporeally, thereby fixing the proximal tip 4 to the guide wire 3 accordingly.

A proximal tip according to the present invention is not particularly limited in form, but it is preferably a nearly elliptical shape to smoothly insert and arrange a stent to a desired position without damaging an internal lumen inner wall. A proximal tip 4 of this embodiment is provided with a proximal tip front end 4b in a nearly pointed form, and a proximal tip rear end 4c in a gradually spherical surface. Hereby, resistance to blood flow can be reduced in insertion and arrangement of the stent 2 into an internal lumen. Meanwhile, in order to withdraw the stent 2 deployed, the proximal tip 4 can be collected from the body without damaging the lumen inner wall.

Next, as shown in Figures 2, 5, 6 and 7, a stent converging and retaining member 5 of this embodiment comprises an operation main body portion 5a and a proximal tip expanded portion 5b. The operation main body portion 5a is composed of wire-like members formed of a longitudinal thin wire to insert a lumen of the stent 2 longitudinally. The proximal tip expanded portion 5b is provided at a proximal end of the operation main body portion 5a formed of plural radially and expandably branched wire-like members. Said proximal tip expanded portion 5b anchors an opening edge part of the stent proximal end opening portion 2a from a lumen of the stent 2 at almost equal intervals, and anchors the stent proximal end opening portion 2a in the proximal tip 4. As a result, the stent proximal end opening portion 2a is converged and retained, thereby controlling the stent by expanding the proximal end thereof irrespective of the control of a stent distal end. In this embodiment, as later mentioned, even when a sheath 6 is pulled to expose the stent 2, only the stent distal end 2b is primarily expanded to deploy the stent.

The stent converging and retaining member 5 is released only by pulling, thereby expanding the stent proximal end opening portion 2a after the stent distal end 2b to deploy the stent 2 entirely. Due to reduction in tensile force on the stent 2 at a minimum level, position shift of the stent 2 deployed can be advantageously eliminated.

As shown in Figures 5 (a) and 6 (a), when the stent converging and retaining member 5 is withdrawn slowly, a stent converging and retaining member 5 whose proximal tip expanded portion 5b is not expanded can be used to cause no damage to an internal lumen inner wall. In this case, the proximal tip expanded portion 5b may be formed of a shape-memory alloy so that it can be expanded slowly. Meanwhile, as shown in Figures 5 (b) and 6 (b), when the stent converging and retaining member 5 is smoothly withdrawn, a stent converging and retaining member 5 whose proximal tip expanded portion 5b is expanded can be employed to smoothly open a stent proximal end opening portion 2a converged and retained. Each stent converging and retaining member 5 can be selected in accordance with way of surgeon's operation and experience.

A stent converging and retaining member according to the present invention is not particularly limited in length as long as a stent which is inserted in an internal lumen and arranged at a desired position can be operated in front of an insertion port.

As shown in Figures 5 and 6, a stent converging and retaining member 5 of this embodiment is formed of a shape-memory material to expand a proximal tip expanded portion 5b as required, but a shape-memory material is not particularly limited and may be e.g., Cu-Al-Ni alloy, Cu-Zn-Al alloy and Ni-Ti alloy such as nitinol.

Next, as shown in Figures 3, 4 and 7, said proximal tip 4 is provided with a stent anchor hole 4a as a stent anchoring portion in this embodiment. The stent 2 is anchored so that a proximal tip expanded portion 5b of a stent converging and retaining member 5 is converged on the stent anchor hole 4a to be engaged with. Specifically, when the proximal tip expanded portion 5b of the stent converging and retaining member 5 anchors the stent proximal end opening portion 2a to be engaged with the stent anchor hole 4a of the proximal tip 4, the proximal tip expanded portion 5b is converged and retained to anchor the stent 2 in the proximal tip 4.

As mentioned above, the proximal tip 4 can delicately control and operate the movement of the stent 2 by operating a guide wire 3, because the proximal tip 4 is fixed to the guide wire 3 and cooperated therewith, and the stent 2 can be guided to a desired position or direction to be arranged.

Since the stent anchor hole 4a is formed in this embodiment, no extra force is imposed on the proximal tip 4 or the stent 2 when the stent converging and retaining member 5 is withdrawn, thereby releasing said distal end opening portion 2a converged and retained and the stent 2 anchored with a small force.

Subsequently, as shown in Figures 1 and 2, a sheath 6 of this embodiment converges the stent 2 to house it therein and moves the stent 2 converged to a desired position. A distal end of the sheath 6 is left in front of an insertion port and a pusher 7 can hold said stent 2 to extract the sheath 6 backward, thereby expanding said stent 2 at a desired position. The sheath 6 is provided with a hemostatic valve 6a at one end to prevent extracorporeal leakage of body fluids or blood flowing into the stent placement device 1.

A sheath 6 of this embodiment is provided with no space with the pusher 7 and a favorable diameter so as to smoothly expose the stent 2.

Next, as shown in Figures 1 and 2, a pusher 7 of this embodiment plays a role in preventing backward position shift of the stent 2 when the sheath 6 is withdrawn. Also, the pusher 7 is arranged so that a proximal end thereof comes into contact with a stent distal end 2b arranged in an internal lumen with a distal end left in front of an insertion port. A pusher 7 of this embodiment is formed so that a distal end left in front of an insertion port is longer than a distal end of the sheath 6 and shorter than a guide wire 3 and a stent converging and retaining member 5.

A pusher 7 of this embodiment is provided with an insertion port 7a which can insert a guide wire 3 and a stent converging and retaining member 5 longitudinally and is formed of a cylindrical portion whose diameter is almost identical to a stent 2 reduced in diameter.

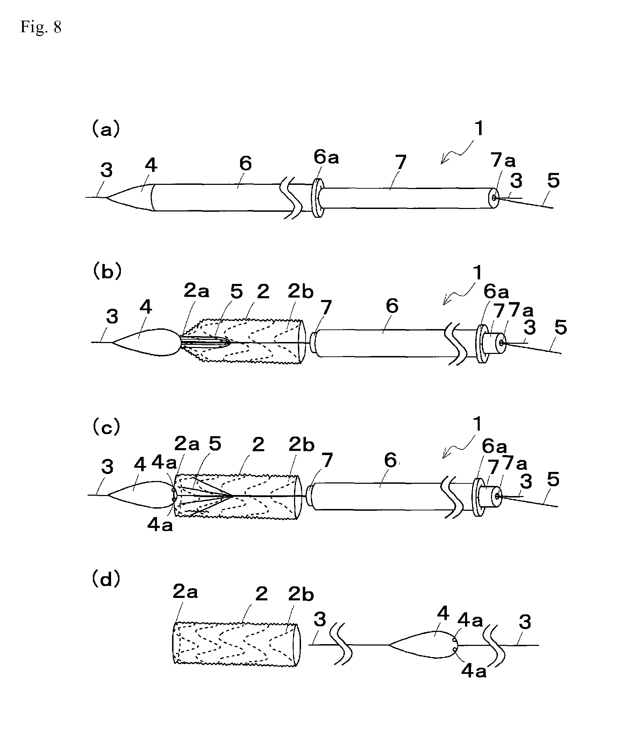

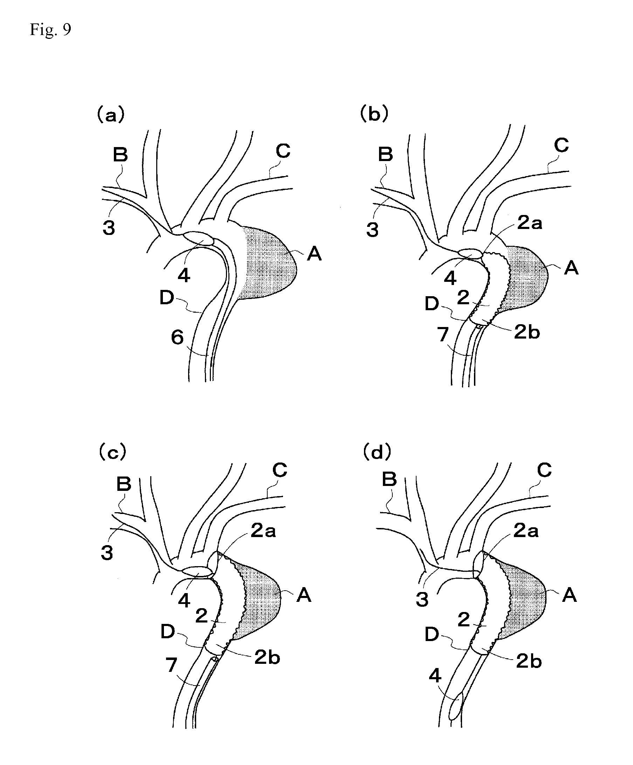

Next, one embodiment of a stent placement method according to the present invention will be described. Figure 8 is an explanatory diagram showing a stent placement method of this embodiment, and Figure 9 is an explanatory diagram showing a stent placement method for deploying a stent in thoracic aneurysm.

A stent placement method of this embodiment, as shown in Figures 8 and 9, is a method for deploying a stent 2 at a desired position in an internal lumen, comprising the following 4 steps.

### [Step 1] Stent deploying step

In a stent placement device 1 integrally set as shown in Figure 8 (a), leading operation of a guide wire 3 inserts the stent 2 into an internal lumen to arrange the stent 2 at a desired position. The stent 2 is anchored in a proximal tip 4, which will be fixed at an intermediate position of the guide wire 3 to cooperate therewith, thereby guiding the stent 2 to a desired position by operating the guide wire 3 and operating and controlling an anchoring position or direction delicately. Then, in the stent 2, a stent proximal end opening portion 2a thereof is converged and retained by a stent converging and retaining member 5 and housed in a longitudinal cylindrical sheath 6 while the stent 2 is entirely reduced in diameter.

For instance, when a stent placement method of this embodiment is employed as a method for treating a thoracic aneurysm A, as shown in Figure 9 (a), a guide wire 3 is inserted from a femoral artery (not shown) into a right brachiocephalic artery B. Then, by operating a distal end of a guide wire 3 left in front of an insertion port of said femoral artery and a proximal end of a guide wire 3 extracorporeally extended from an extraction port of said right brachiocephalic artery B, a stent proximal end opening portion 2a is arranged at an arcus aortae section distally immediately beneath a left subclavian artery C to prevent left subclavian artery C from clogging. Here, "proximal" means being close to the heart and "distal" means being distant from the heart.

### [Step 2] First stent deploying step

Next, as shown in Figure 8 (b), as a distal end of the pusher 7 is pushed to hold the stent 2, the sheath 6 is withdrawn toward a distal end thereof, and the stent 2 housed with reduced in diameter is exposed and expanded. Then, since a stent proximal end opening portion 2a is restrained and retained by a stent converging and retaining member 5, only a stent distal end 2b expands to be deployed at a desired position in an internal lumen.

Here, in a case where said thoracic aneurysm A is treated, as shown in Figure 9 (b), operation of the sheath 6 and the pusher 7 left in front of an insertion port of a femoral artery withdraws the sheath 6 to deploy a stent distal end 2b in a descending aorta upper portion D distal to a thoracic aneurysm A.

### [Step 3] Second stent deploying step

Subsequently, as shown in Figure 8 (c), a stent converging and retaining member 5 is withdrawn to release a stent proximal end opening portion 2a and expand a proximal end of the stent, thereby entirely deploying the stent 2 at a desired position in an internal lumen.

In a case where said thoracic aneurysm A is treated, as shown in Figure 9 (c), operation of a stent converging and retaining member 5 left in front of an insertion port of a femoral artery releases a stent proximal end opening portion 2a to deploy the stent 2 so as to bridge a thoracic aneurysm A. Thus, a new blood vessel cavity can be formed around the thoracic aneurysm A, using the stent 2.

### [Step 4]

Thereafter, as shown in Figure 8 (d), the guide wire 3 is withdrawn to collect the guide wire 3 and a proximal tip 4 fixed thereto from the body. In this embodiment, a proximal tip rear end 4c is formed of a gradually curved shape. Due to a small diameter of the stent placement device 1, a proximal tip 4 can be collected from the body without damaging an internal lumen inner wall. Subsequently, the sheath 6 and the pusher 7 left in the body are completely withdrawn from the body.

In a case where said thoracic aneurysm A is treated, as shown in Figure 9 (d), by pulling a guide wire 3 left in front of an insertion port of a femoral artery, a stent placement device 1 other than a stent 2 can be collected from an internal lumen.

Specifically, in a stent placement method of this embodiment, Step 1 arranges a stent 2 at a desired position, Step 2 deploys a stent distal end 2b and Step 3 deploys a stent proximal end opening portion 2a, resulting in placement of the entire stent 2 and collection of a proximal tip 4, as well as a guide wire 3, by Step 4.

Step 1 is operated to place a stent 2 in the body, and Steps 2 to 4 "withdraw or pull" the stent. For example, a thoracic aneurysm, in which a patient is conventionally subjected to cardiac arrest state in stent treatment, can be treated under non-cardiac arrest, or if any, under significantly short period of cardiac arrest.

According to this embodiment above mentioned, the following advantages are obtained.
1. Since a stent can be expanded primarily in a distal end and then in a proximal end to deploy the stent in an internal lumen, stent position shift due to blood flow can be prevented and the stent can be correctly arranged at a desired position.
2. Operation of a guide wire only can delicately operate and control a stent.
3. A stent placement device can be smoothly inserted into an internal lumen without being got stuck at a bent section, and significantly low-invasiveness treatment can be provided without damaging an internal lumen inner wall.
4. Only by withdrawing a guide wire, a proximal tip can be surely collected.
5. Due to few components and simple structure in a stent placement device, in addition to few and easy stent placement procedures, the stent placement device can be smoothly operated without operational errors.

A stent placement method according to the present invention is not limited to this embodiment, but may be altered accordingly.

### EXPLANATION OF LETTERS OR NUMERALS

- 1: Stent placement device
- 2: Stent
- 2a: Stent proximal end opening portion
- 2b: Stent distal end
- 3: Guide wire
- 4: Proximal tip
- 4a: Stent anchor hole
- 4b: Proximal tip front end
- 4c: Proximal tip rear end
- 5: Stent converging and retaining member
- 5a: Operation main body portion
- 5b: Proximal tip expanded portion
- 6: Sheath
- 6a: Hemostatic valve
- 7: Pusher
- 7a: Insertion port
- A: Thoracic aneurysm
- B: Right brachiocephalic artery
- C: Left subclavian artery
- D: Descending aorta upper portion

## Claims

1. A stent placement device for deploying a stent in an internal lumen, comprising:
a guide wire which guides said stent to a desired position in an internal lumen;
a proximal tip which is fixed at an intermediate position of said guide wire and provided with a stent anchoring portion for anchoring said stent; and
a stent converging and retaining member which converges and retains a stent proximal end opening portion of said stent.

2. The stent placement device as set forth in Claim 1, wherein said stent converging and retaining member comprises an operation main body portion formed of a longitudinal thin wire and a proximal tip expanded portion composed of plural thin linear members which are radially and expandably branched at a proximal tip of the operation main body portion.

3. The stent placement device as set forth in Claim 1 or Claim 2, wherein said stent converging and retaining member is formed of a shape-memory material.

4. The stent placement device as set forth in any one of Claims 1 to 3, wherein said stent anchoring portion is composed of one or plural anchor holes, and said proximal tip expanded portion of said stent converging and retaining member is engaged with said anchor hole to be anchored.

5. A stent placement method for deploying a stent at a desired position in an internal lumen, comprising:
a stent arranging step for arranging a stent at a desired position by restraining and retaining a proximal end opening portion of a stent reduced in diameter by a stent converging and retaining member, anchoring the stent in a proximal tip fixed at an intermediate position of a guide wire and inserting the stent into an internal lumen with the stent being housed in a longitudinal tube-shaped sheath and guiding the stent by said proximal tip;
a 1st stent deploying step for deploying only a stent distal end at a desired position by pulling said sheath toward a distal end to expand the distal end of said stent with a proximal end opening portion of said stent restrained and retained by said stent converging and retaining member;
a 2nd stent deploying step for entirely deploying the stent at a desired position by releasing the proximal end opening portion by said stent converging and retaining member; and
a step for collecting said proximal tip from the body by withdrawing said guide wire from an internal lumen.
